# EUROPEAN PATENT SPECIFICATION

(11) **EP 1 439 482 B1**
(45) Date of publication and mention of the grant of the patent: **05.09.2007**
(21) Application number: 04250170.0
(22) Date of filing: 15.01.2004
(51) Int. Cl.: G06F 19/00

(54) **System for controlling medical data acquisition processes**
System zur Steuerung von medizinischen Datenerfassungsprozessen
Système pour contrôler les opérations de saisie de données médicales

(30) Priority: 15.01.2003 GB 0300922
(43) Date of publication of application: 21.07.2004
(73) Proprietor: Mirada Solutions Ltd, Oxford OX1 2ET (GB)
(72) Inventor: Behrenbruch, Christian Peter, Oxford OX1 2ET (GB); Declerck, Jerome Marie Joseph, Oxford OX1 2ET (GB); Brady, John Michael, Oxford OX1 2ET (GB)
(74) Representative: Nicholls, Michael John

(56) References cited:
- WO-A-00/57361
- US-A- 5 590 654
- US-A1- 2002 068 865
- US-B1- 6 397 097
- US-B1- 6 408 201

## Description

The present invention relates to a system for controlling the acquisition of medical data, in particular to provide for the possibility of improved medical data acquisition protocols.

The acquisition of medical data such as image or spectroscopy data is an important part of modem medical practice. As diagnostic imaging (medical imaging) with technologies such as Magnetic Resonance Imaging (MRI), Computed Tomography (CT) and Positron Emission Tomography (PET) increase in spatial resolution, acquisition speed and clinical flexibility, sophisticated diagnostic techniques involving the injection of imaging agents to characterise very specific physiological and pathophysiological indicators have evolved. This concept extends from very basic contrast agents (for example, iodine blood pool agents that increase the x-ray attenuation of a CT scan and hence improve the contrast of blood vessels) to the new fields known as "molecular imaging" and "functional imaging".

Molecular imaging (MI) and functional imaging (FI) are revolutionary because imaging agents can be used which target very specific biophysical, cellular or genetic behaviour. It is possible to screen for a specific disease by attaching a gene to an imaging agent that only makes the compound visible during medical imaging if a particular disease (e.g. oncogene) is present. This notion extends to a vast range of applications from oncology to cardiology and neuroimaging for diseases such as Parkinson's and Alzheimer's syndromes.

Given that molecular imaging (which can be broadly generalised to "functional imaging") can accurately characterise a disease state, it can also accurately characterise treatment response to surgery, chemotherapy (including gene therapy, stem cell therapy) and radiotherapy. However in order to benefit fully from such functional imaging techniques it would be advantageous to achieve the control and measurement of delivery of the imaging agent. Even slight variations in the injection profile or inhalation of a highly sophisticated imaging agent can alter the measured value of the indicated [patho]phyisiology. This is important calibration information for the analysis of the imaging process.

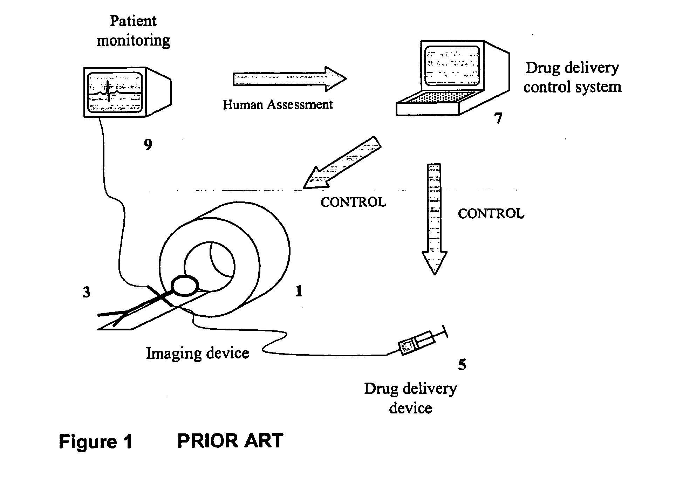

Currently some devices exist to control and trigger drug delivery and image acquisition. This is illustrated in Figure 1 of the accompanying drawings. A medical data acquisition device 1, in this case an imaging device, is operable to provide images of a subject 3, in this case a human patient. During the image acquisition process a drug delivery device 5 is used to administer a contrast agent to the patient and the drug delivery and image acquisition are synchronised in accordance with a predefined acquisition protocol under control of a computer-based control system 7. Conventionally the patient's condition may be monitored (such as blood pressure, ECG etc) by an additional patient monitoring device schematically illustrated as 9. A human operator of the system may abort the acquisition protocol if the patient monitoring device 9 shows an undesirable change in the patient's condition.

However, this conventional type of system can only put into practice a set, pre-defined acquisition protocol. The only way of adapting the protocol is in response to the human assessment of the patient's condition. With many acquisition techniques this can cause problems because the timing of the image acquisition should be responsive to the behaviour of the agent in the subject, rather than simply to the timing of the command to the drug delivery device.

Similar problems arise in other medical data acquisition techniques such as spectroscopy (e.g. magnetic resonance spectroscopy).

US-A-2002-0068865, on which the pre-characterising portion of claim 1 is based discloses a system similar to that above, but in which the drug delivery device can be controlled by the detection system or imaging controller, so that the imaging can be synchronized with a peak contrast in the imaged area.

According to the present invention there is provided a system as defined in claim 1.

Preferably the data is processed in "real time", i.e. as it arrives, or within a short time compared to the timing of the whole procedure, so that "real time" control of the data acquisition apparatus based on the processed results is then possible.

Thus the present invention allows synchronisation of the agent administration (such as an imaging agent, e.g. contrast agent) with the results of the data acquisition itself (e.g. the image being acquired). This allows a much closer control of the acquisition protocol.

The device for administering the agent may be an automatic drug delivery device, and the agent may be a pharmaceutical agent, such as dobutamine for stress heart imaging, or an imaging agent, such as a contrast agent, in the case of medical imaging.

The protocol controller may control the type and amount of agent administered so that, for example, more than one type of agent may be administered at different times through the protocol.

The protocol controller may also be responsive to additional data acquired from the subject independently of the medical data acquisition apparatus. For example, in the case of a human or animal subject this may be in response to patient movement, blood pressure, heart rate variation etc. In particular, to successfully acquire medical images, particularly for moving organs, it is sometimes necessary to include physiological measurements of the patient, such as heart rate/characteristics, respiratory information, motion etc. In one specific case this could be as simple as recording head movement during an MR scan, which movement introduces artefacts in the image, which could be confused with disease. The protocol controller can, for example, suspend image acquisition temporarily during a period of patient movement, thus adapting the protocol in real time.

The system is applicable both to the acquisition of medical data *in vivo,* e.g. from a human, animal or plant subject, and *in vitro,* e.g. from medical samples. It is applicable to dynamic studies, i.e. the use of medical imaging to characterise the physiological or pathophysiological behaviour as a function of time. Often this is performed in conjunction with an *in vivo* imaging agent which has specific biophysical or pharmacokinetic behaviour. The invention is also applicable to pharmacokinetics, i.e. the biophysical characterisation of a molecule, compound, gene etc in or from the body.

The invention allows the synchronisation of the agent delivery and data acquisition, including baseline acquisition and delays between the delivery of the agent, its delivery profile and the commencement of acquisition. Additionally, with knowledge (e.g. a model) of an agent's behaviour in the subject, it is possible to optimise the way in which the acquisition process is conducted with respect to the subject, for example in the case of a human or animal, in response to the height, weight, cardiac throughput of the subject, or the characteristics of the data acquisition apparatus itself. This can give a better managed acquisition, better image quality, an optimised tradeoff between spatial and temporal resolution and, in certain cases (for example, with CT) minimisation of radiation dosed to the patient.

Synchronisation of the analysis of the data acquisition with the agent delivery allows critical timing information to be obtained to optimise the information produced by the interaction between the agent and the physiological phenomenon under investigation. Further, real time analysis of the behaviour of the agent can be used to alter the acquisition sequence in order further to improve the acquisition result, reduce false positives, negatives, artefacts, and to calibrate the analysis of the agent against fluctuations in other measured parameters of subject, such as blood pressure, respiration rate etc in a human or animal.

The invention will be further described by way of example with reference to the accompanying drawings in which:-
Figure 1 illustrates a prior art medical data acquisition system;
Figure 2 illustrates a medical data acquisition system in accordance with one embodiment of the present invention;
Figure 3 illustrates the critical process timings in an image acquisition process for liver CT imaging in accordance with an embodiment of the present invention;
Figures 4(a) to (f) illustrate a frame sequence showing the change in intensity with time in a Gadolinium enhanced MRI scan; and
Figure 5 illustrates the change in intensity of a given voxel over time in a typical Gadolinium enhanced MRI scan.

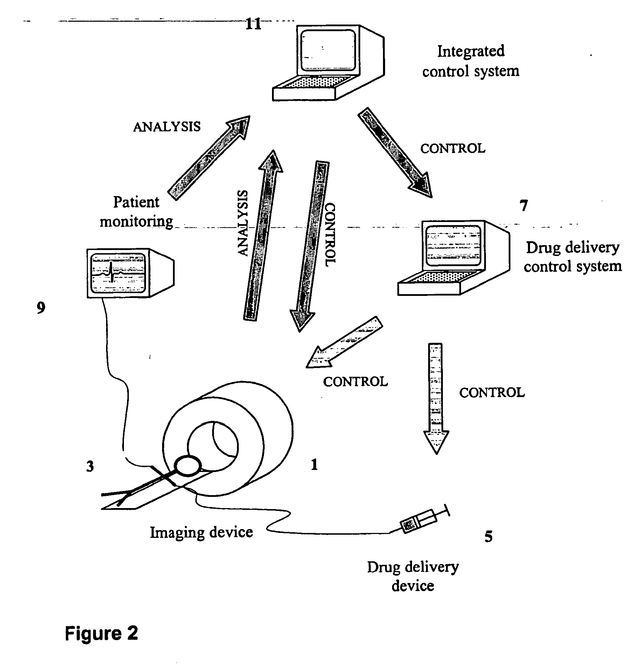

Figure 2 illustrates a control system in accordance with the present invention applied to the imaging apparatus described above with reference to Figure 1. The parts found in Figure 1 are labelled with the same reference numerals and thus include the imaging device 1, the subject 2, the drug delivery device 5, the control system 7 and the additional patient monitor 9. In accordance with the present invention, though, the acquisition device 1 and agent administering device 5 are controlled, via the control system 7, by a supervisory control system 11. While this is shown separately from the control system 7 in Figure 2, the two may be combined. The supervisory control system 11 receives data from the imaging device and also from the additional patient monitor 9 and controls the drug delivery device 5 and imaging device 1 in real time in response to this image data and additional data from the monitoring device 9. Thus the supervisory control device 9 connects the imaging agent (drug) delivery mechanism 5, the imaging process, the acquisition of physiological parameters from the patient via monitoring device 9 and the analysis of the behaviour of the imaging agent for the purposes of diagnosis. It is illustrated in Figure 2 as being embodied in a general purpose personal computer, i.e. as a single integrated software system, though it can be embodied as a distributed set of software tools across the control system 7, drug delivery device 5, imaging device 1 and patient monitor 9. The supervisory controller 11 is effective via the control system 7 to control both the relative timings of drug delivery and image acquisition, and the type of drug (e.g. contrast agent) delivered and the mode of data acquisition. Thus multiple imaging agents may be administered with a desired administration profile, and the imaging device may be controlled accordingly, to switch modes in accordance with the different agents administered.

Two different specific examples of the type of control made possible by the present invention will now be described.

### 1) CT Imaging of the Liver

Conventionally, liver tumours are assessed with CT images enhanced with contrast agents. The agent is used to create contrast between vascularisation and the tissue itself. The objective of the diagnostic based on such images is two-fold:
1) detection and assessment of liver tumours. Tumours are characterised by lower contrast compared to the surrounding healthy tissue when the contrast agent washes in through the portal vein. Some other imaging modality like PET can be used to differentiate cancerous tumours from cysts or necrosis and degeneration from cirrhotic diseases, for instance. The main use of the CT image is to determine the location and extent of the tumours.
2) visualisation of the vascularisation of great vessels for surgery planning. When surgery is planned for the resection of the tumours, great care must be taken to remove the minimum amount of liver. The clinician uses the vascularisation tree to determine the region to remove.

The selection of the resection region is a complex process: in clinical procedures, the liver is decomposed into 8 different segments, where boundaries are based on anatomical landmarks (Lafortune et al., "Segmental anatomy of the liver: a sonographic approach to the Couinaud nomenclature":Radiology 181(2):443-448, 1991). Other approaches enhance the precision of the definition of these boundaries using the portal vein instead of external shape-based landmarks (L. Soler, H. Delingette, G. Malandain, J. Montagnat, N. Ayache, C. Koehl, O. Dourthe, B. Malassagne, M. Smith, D. Mutter and J. Marescaux: "Fully automatic anatomical, pathological, and functional segmentation from CT scans for hepatic surgery", Computer Aided Surgery, vol. 6, num. 3, August 2001). Basing the segmentation of liver on vascular tree is absolutely key for surgery planning, as the vascularisation of the liver is one of the most complex of the whole human body. There are so many blood vessels that a cut must minimise bleeding. Cutting through a great vessel is simple, as bleeding is easily controlled with clamping. Cutting through capillaries is simple as well, as cauterisation stops bleeding. But cutting at random in the liver can cause the rupture of many intermediate sized vessels, which cannot be cauterised because they are too big, and for which clamping is not practical because hundreds of such vessels could be opened in one single cut.

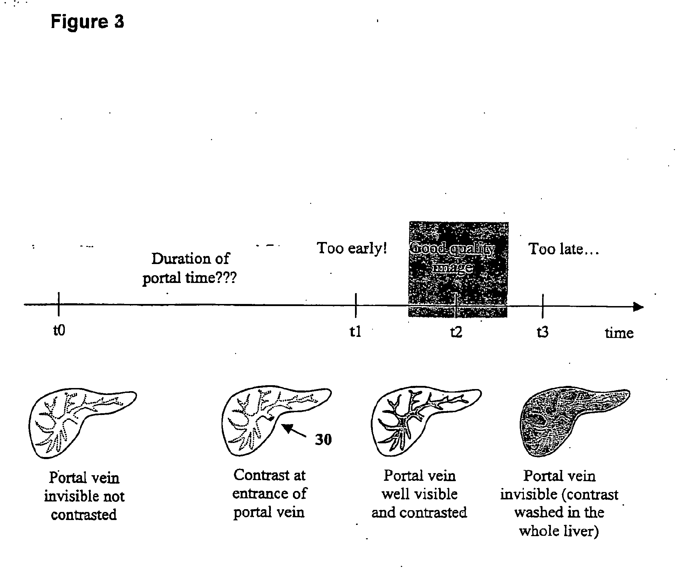

Identification of the segments based on vascularisation is a more and more clinically accepted practice, but it needs good vascular tree segmentation. Such segmentation can be obtained for instance using CT imaging as illustrated in Figure 3. In a normal CT scan, the portal tree is not visible nor differentiable from surrounding liver cells, so the use of a contrast agent is necessary. Following a classic imaging protocol, the CT image is acquired very quickly after some contrast agent has been delivered into the patient's blood. The contrast agent is injected in a single bolus (t0), and after a small period of time (t1, which is called *portal time*), the bolus reaches the portal vein 30, one of the main vascular trees irrigating the liver. The contrast agent washes into the liver and enhances all vascular branches, until it reaches the capillaries (t2). Then, contrast agent washes in all cells and other artery trees before flowing out in the vena cava (t3). By this time, the whole liver is brightened by the contrast agent and the portal tree is no longer visible.

There are two main difficulties in the acquisition of a good and usable image:
1) The image needs to be acquired at t2, and not at t1 or t3. The problem is that the margin t3-t1 is very small, typically 5 or 10 seconds, so the image must be acquired very quickly.
2) The portal time needs to be estimated but cannot be determined a priori with accuracy. Usually, t1-t0 is between 10 and 20 seconds. If the portal time t1 is not estimated correctly, the image is unusable, either too dark, or too bright, with no contrasting vascular tree.

So the evaluation of the exact portal time, i.e. time point t₁, is the key to triggering the start of the imaging procedure. With the present invention the protocol controller receives the data from the image acquisition apparatus and so is programmed to look for the arrival of contrast agent at the entrance 30 to the portal vein. On detection on the image of contrast agent at the portal vein the time point t1 is established with certainty. Thus rather than estimating the time for contrast agent to arrive from the drug delivery device to the portal vein (which could be about 20 seconds), its exact arrival at the entrance to the portal vein is detected. Then an image acquisition protocol for obtaining different slice images through the liver can be started to cover the short time period, for example 5 to 10 seconds, between times t1 and t3. Automatic detection of the arrival of the contrast agent at the portal vein is achieved, for example, by the clinician first determining the location of the entrance of the portal vein in a CT "scout" image used for setting a region of interest for the 3D image acquisition. The scanner then acquires at regular time intervals (e.g. every 2 or 3 seconds) an image of the area, and compares each image with the preceding one. The arrival of contrast agent shows up easily as a bright area on the previously uniformly grey vessel.

### MR Imaging of the Breast

An example based on an optimised contrast-enhanced magnetic resonance image of the breast will now be described.

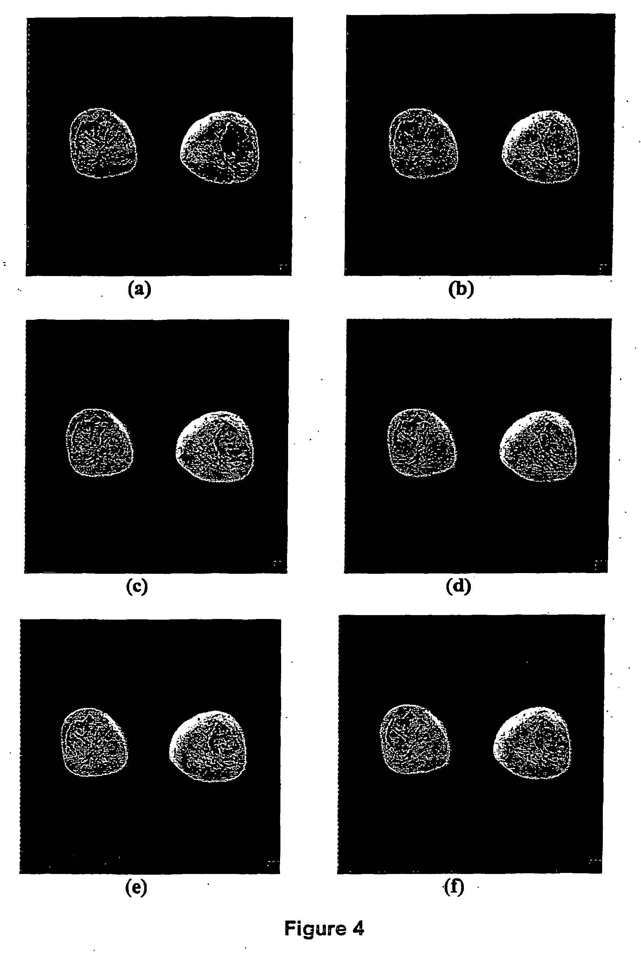

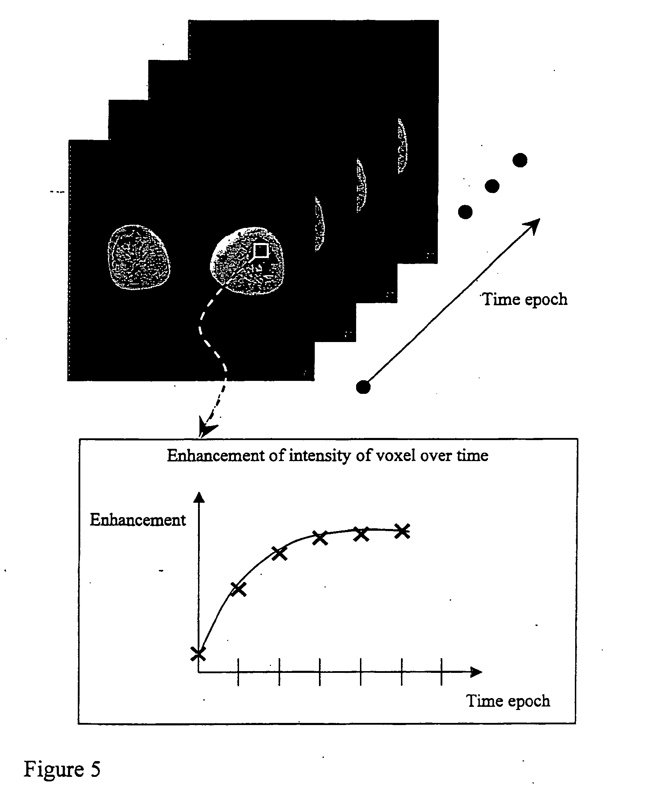

Figures 4 and 5 illustrate the typical dependency of contrast enhanced intensity against time using Gadolinium (Gd) as a contrast agent. Figure 4 illustrates six frames of the sequence and Figure 5 shows a graph of the intensity of a given voxel. It can be seen that the intensity arises quite steeply over the first one or two minutes, e.g. through images (a), (b) and (c), and then decays very gradually, little change ocurring from images (c) through (f). In order to accurately characterise the nature of the material being enhanced it is necessary to detect the shape of this curve as accurately as possible. In particular, for example, it is important to be able to detect the peak intensity. Rather than following an acquisition protocol based on estimated behaviour, which may be based on some particular tissue type (or some average) the present invention allows different image acquisition modalities to be used during different times in the protocol. For example:-
1) The supervisory control system 11 invokes a "baseline" (pre-injection) image acquisition along with calibration images for various physics-based corrections. For example, the paper Armitage P.A., Behrenbruch C.P., Brady, J.M. and Moore N. "Optimising flip-angles for improved T1 calculation in 3D contrast-enhanced breast MR imaging" in Proc. International Society of Magnetic Resonance in Medicine 2001 p. 526, describes a protocol to acquire images to compute a T1 map, which is characteristic of the tissue. (There are two reasons why a contrast agent shows a bright spot in MRI: (a) the T1 of the tissue is high, and becomes higher when the high-T1 contrast agent washes in, and (b) the tissue has an average T1, but there is a lot of contrast agent washing in. The difference is (a) is indicative of tissue that is definitely benign and normal, whereas (b) could be indicative of the trace of malignant tumour. Basically, when looking at the post-contrast image, there is no way to be able to quantitate how much of the brightening effect comes from the contrast agent and how much comes from the tissue. Obtaining a T1 map prior to the acquisition allows correction of the enhancement map for these artefacts.)
2) It then commands the delivery device 5 to inject Gadolinium (Gd) in a controlled bolus injection profile to be imaged in step (3) and characterised as an arterial input function in preparation for analysis.
3) The supervisory control system 11 immediately switches the MRI system 1 to a rapid echoplaner (EPI sequence) which performs a low spatial, high temporal resolution image (1 second acquisition) of the region of interest in the breast (or, in other examples, different parts of the body, e.g. the brain) until the measured contrast-induced (Gd) signal intensity in the imaging process reaches 100% of baseline. This period is critical for characterising regions of angiogenesis indicated by the uptake of gadolinium.
4) The supervisory control system 11 then switches the acquisition profile of the MRI system 1 to a slower, high-resolution gradient echo (e.g. Fast SPoiled GRadient echo pulse sequence - FSPGR) sequence that acquires high quality 3D volumes to measure the plateau of contrast agent behaviour. Simultaneously, a smaller steady infusion of Gd is added via the delivery device 5 to buffer the peak contrast.
5) A series of FSPGR sequences are acquired until the washout is sufficiently characterised to end acquisition. At the point where the analysis tools have all the phamacokinetic parameters needed, acquisition is ceased.
6) The user is presented with a finalised analysis of the MRI data showing accurately parameterised areas of uptake which statistically correspond to regions indicative of tumour angiogenesis. This result is optimised as the analysis tool was "in time" to detect different phases of the contrast agent's biophysical behaviour (including injection profile) which in turn controlled infusion of Gd and the acquisition protocol.

The invention can also accept data input from other sources, such as a heart rate monitor 9. Thus, for example, if midway through the acquisition the patient experiences some anxiety, as detected by an increase in cardiac activity, and moves, the supervisory protocol controller 11 can suspend acquisition, optionally motion correct to the new patient geometry (e.g. using the technique described in WO 00/57361) and continue with the acquisition process.

Although the invention has been described above with respect to specific imaging techniques, it is applicable to other imaging and data acquisition techniques including the use of: medical imaging by devices such as CT/MRI/PET/SPECT/X-ray/ Ultrasound/Optical, functional imaging, dynamic imaging, imaging with radiopharmaceuticals, contrast agents, hyperpolarised gases, x-ray attenuation agents, genetic/oncogenic markers, cellular/receptor level indicators, blood pool/flow agents and combined targeted imaging agents/targeted therapy agents.

The invention may be embodied in a computer program which, when executed on a general purpose computer, controls the data acquisition and agent administering device. Alternatively it may be embodied in firmware, programmable logic or dedicated process controls and automation systems.

## Claims

1. A system for controlling a medical image data acquisition process, comprising an image data acquisition apparatus controller (11) for controlling image data acquisition apparatus to acquire medical image data from a subject (3);
a contrast agent administration controller (7) for controlling a device (5) to administer a contrast agent to the subject (3);
a medical image data processor (11) for receiving the medical image data from the image data acquisition apparatus and processing it;
a protocol controller (11) for controlling the image data acquisition apparatus controller (11) and the contrast agent administration controller (7) in response to data from the medical image data processor (11); wherein:
the medical image data processor (11) is adapted to process the medical image data to detect different phases of the contrast agent's behaviour in the subject (3) and in turn to cause the protocol controller (11) to control the contrast agent administration controller (7) to change the amount of contrast agent administered to the subject (3) and simultaneously to control the image data acquisition apparatus controller (11) to change the mode of image data acquisition to acquire image data in at least two different modes; **characterised in that**:
the medical image data processor (11) is adapted to perform real time analysis of the behaviour of the contrast agent in the subject to characterise the uptake and washout of contrast agent thereby to provide pharmacokinetic parameters of the behaviour of the contrast agent, and to cease acquisition when the medical image data processor (11) has all the pharmacokinetic parameters needed; and **in that** said controller (11) is adapted to control the acquisition apparatus to perform said change of mode of image data acquisition to provide an optimized tradeoff between spatial and temporal resolution in said different phases of the contrast agent's biophysical behaviour.

2. A system according to claim 1 wherein the device (5) to administer a contrast agent to the subject (3) is an automatic drug delivery device (5).

3. A system according to claim 1 or 2 wherein the contrast agent is a pharmaceutical agent.

4. A system according to any one of the preceding claims wherein the image data acquisition apparatus is a spectroscopy apparatus and the medical image data processor (11) is a spectral data processor for processing data acquired by the spectroscopy apparatus.

5. A system according to any one of the preceding claims wherein the protocol controller (11) is adapted to control the contrast agent administration controller (7) to control the type of contrast agent administered to the subject (3) in response to processed medical data results from the medical image data processor (11).

6. A system according to any one of the preceding claims wherein the protocol controller (11) is adapted to be responsive to additional data acquired from the subject (3) independently of the medical image data acquisition apparatus.

7. A system according to any one of the preceding claims adapted to acquire the medical image data *in vivo* from a human, animal or plant as said subject (3).

8. A system according to any one of claims 1 to 8 adapted to acquire the medical image data *in vitro.*

## Patentansprüche

1. System zum Regeln eines medizinischen Bilddatenerfassungs-Verfahrens, umfassend einen Controller für ein Bilddatenerfassungsgerät (11) zum Regeln eines Bilddatenerfassungsgeräts zum Erfassen von medizinischen Bilddaten von einem Subjekt (3);
einen Controller für die Kontrastmittelverabreichung (7) zum Regeln einer Vorrichtung (5) zum Verabreichen eines Kontrastmittels an das Subjekt (3);
einen Prozessor für medizinische Bilddaten (11) zum Empfangen der medizinischen Bilddaten von dem Bilddatenerfassungsgerät und zu deren Verarbeitung;
einen Protokoll-Controller (11) zum Regeln des Controllers für ein Bilddatenerfassungsgerät (11) und des Controllers für die Kontrastmittelverabreichung (7) als Antwort auf Daten von dem Prozessor für medizinische Bilddaten (11); wobei:
der Prozessor für medizinische Bilddaten (11) dafür eingerichtet ist, die medizinischen Bilddaten zu verarbeiten, um verschiedene Phasen des Verhaltens des Kontrastmittels in dem Subjekt (3) zu erkennen und in Folge den Protokoll-Controller (11) zu veranlassen, den Controller für die Kontrastmittelverabreichung (7) zum Ändern der Menge an Kontrastmittel, die dem Subjekt (3) verabreicht wird, zu regeln und gleichzeitig den Controller für ein Bilddatenerfassungsgerät (11) zum Ändern des Modus der Bilddatenerfassung zum Erfassen von Bilddaten in wenigstens zwei verschiedenen Modi zu regeln; **dadurch gekennzeichnet, dass**:
der Prozessor für medizinische Bilddaten (11) dafür eingerichtet ist, eine Echtzeit-Analyse des Verhaltens des Kontrastmittels in dem Subjekt durchzuführen, um die Aufnahme und das Ausspülen von Kontrastmittel zu charakterisieren und so pharmakokinetische Parameter des Verhaltens des Kontrastmittels bereitzustellen, und um die Erfassung zu beenden, wenn der Prozessor für medizinische Bilddaten (11) über alle benötigten pharmakokinetischen Parameter verfügt; und dass der Controller (11) dafür eingerichtet ist, das Erfassungsgerät zu regeln, um das Ändern des Modus der Bilddatenerfassung durchzuführen, um einen optimierten Kompromiss zwischen räumlicher und zeitlicher Auflösung bei den verschiedenen Phasen des biophysikalischen Verhaltens des Kontrastmittels zu liefern.

2. System nach Anspruch 1, wobei die Vorrichtung (5) zum Verabreichen eines Kontrastmittels an das Subjekt (3) eine automatische Arzneimittel-Verabreichungsvorrichtung (5) ist.

3. System nach Anspruch 1 oder 2, wobei das Kontrastmittel ein pharmazeutisches Mittel ist.

4. System nach einem der vorangegangenen Ansprüche, wobei das Bilddatenerfassungsgerät ein spektroskopisches Gerät ist und der Prozessor für medizinische Bilddaten (11) ein Prozessor für Spektraldaten zum Verarbeiten von Daten, die von dem spektroskopischen Gerät erfasst worden sind, ist.

5. System nach einem der vorangehenden Ansprüche, wobei der Protokoll-Controller (11) dafür eingerichtet ist, den Controller für die Kontrastmittelverabreichung (7) zu regeln, um die Art des Kontrastmittels, das dem Subjekt (3) verabreicht wird, als Antwort auf verarbeitete medizinische Datenergebnisse des Prozessors für medizinische Bilddaten (11) zu regeln.

6. System nach einem der vorangehenden Ansprüche, wobei der Protokoll-Controller (11) so eingerichtet ist, dass er auf zusätzliche Daten, die unabhängig von dem medizinischen Bilddatenerfassungsgerät von dem Subjekt (3) erfasst wurden, anspricht.

7. System nach einem der vorangegangenen Ansprüche, das zum Erfassen der medizinischen Bilddaten *in vivo* von einem Menschen, einem Tier oder einer Pflanze als Subjekt (3) eingerichtet ist.

8. System nach einem der Ansprüche 1 bis 8, das zum Erfassen der medizinischen Bilddaten *in vitro* eingerichtet ist.

## Revendications

1. Système de contrôle d'un processus d'acquisition de données d'images médicales, comprenant un contrôleur d'appareil d'acquisition de données d'images (11) pour contrôler un appareil d'acquisition de données d'images de façon à acquérir des données d'images médicales à partir d'un sujet (3) ;
un contrôleur d'administration d'agent de contraste (7) pour contrôler un dispositif (5) de façon à administrer un agent de contraste au sujet (3) ;
un processeur de données d'images médicales (11) pour recevoir les données d'images médicales de la part de l'appareil d'acquisition de données d'images et à les traiter ;
un contrôleur de protocole (11) pour contrôler le contrôleur d'appareil d'acquisition de données d'images (11) et le contrôleur d'administration d'agent de contraste (7) en réponse aux données provenant du processeur de données d'images médicales (11); dans lequel :
le processeur de données d'images médicales (11) est adapté pour traiter les données d'images médicales pour détecter des phases différentes du comportement de l'agent de contraste dans le sujet (3) et à amener le contrôleur de protocole (11) à contrôler le contrôleur d'administration d'agent de contraste (7) pour changer la quantité d'agent de contraste administrée au sujet (3) et pour contrôler simultanément le contrôleur d'appareil d'acquisition de données d'images (11) pour changer le mode d'acquisition de données d'images pour acquérir des données d'images dans au moins deux modes différents ; **caractérisé en ce que** :
le processeur de données d'images médicales (11) est adapté pour effectuer une analyse en temps réel du comportement de l'agent de contraste dans le sujet afin de caractériser l'administration et la sortie de l'agent de contraste pour fournir des paramètres pharmacocinétiques du comportement de l'agent de contraste, et pour cesser l'acquisition lorsque le processeur de données d'images médicales (11) possède l'ensemble des paramètres pharmacocinétiques nécessaires; et **en ce que** ledit contrôleur (11) est adapté pour contrôler l'appareil d'acquisition pour effectuer ledit changement de mode d'acquisition de données d'images afin d'assurer un compromis optimisé entre la résolution spatiale et la résolution temporelle dans lesdites différentes phases du comportement biophysique de l'agent de contraste.

2. Système selon la revendication 1, dans lequel le dispositif (5) pour administrer un agent de contraste au sujet (3) est un dispositif de délivrance de médicaments automatique (5).

3. Système selon la revendication 1 ou 2, dans lequel l'agent de contraste est un agent pharmaceutique.

4. Système selon l'une quelconque des revendications précédentes, dans lequel l'appareil d'acquisition de données d'images est un appareil de spectroscopie et le processeur de données d'images médicales (11) est un processeur de données spectrales pour traiter les données acquises par l'appareil de spectroscopie.

5. Système selon l'une quelconque des revendications précédentes, dans lequel le contrôleur de protocole (11) est adapté pour contrôler le contrôleur d'administration d'agent de contraste (7) de façon à contrôler le type d'agent de contraste administré au sujet (3) en réponse aux résultats des données médicales traitées provenant du processeur de données d'images médicales (11).

6. Système selon l'une quelconque des revendications précédentes, dans lequel le contrôleur de protocole (11) est adapté pour réagir à des données supplémentaires acquises à partir du sujet (3) indépendamment de l'appareil d'acquisition de données d'images médicales.

7. Système selon l'une quelconque des revendications précédentes, adapté pour acquérir les données d'images médicales in vivo à partir d'un être humain, d'un animal, ou d'une plante en tant que ledit sujet (3).

8. Système selon l'une quelconque des revendications 1 à 8, adapté pour acquérir les données d'images médicales in vitro.
